(19) Europäisches Patentamt
European Patent Office
Office européen des brevets

(11) Publication number: 0 058 072
A2

# (12) EUROPEAN PATENT APPLICATION

(21) Application number: 82300605.1

(22) Date of filing: 08.02.82

(51) Int. Cl.³: C 07 D 213/65
//C07D491/04, (C07D491/04, 319/00, 221/00)

(30) Priority: 09.02.81 US 232923

(43) Date of publication of application: 18.08.82 Bulletin 82/33

(84) Designated Contracting States: AT BE CH DE FR GB IT LI LU NL SE

(71) Applicant: PFIZER INC.
235 East 42nd Street
New York, N.Y. 10017(US)

(72) Inventor: Cue, Berkeley Wendell, Jr.
266 Stoddards Wharf Drive
Gales Ferry Connecticut(US)

(72) Inventor: Massett, Stephen Sargent
78 Brandegee Avenue
Groton Connecticut(US)

(74) Representative: Wood, David John et al,
Pfizer Limited Ramsgate Road
Sandwich Kent CT13 9NJ(GB)

## (54) Process and intermediates for preparing pirbuterol and analogs.

(57) A process for the preparation of a compound of the formula:-

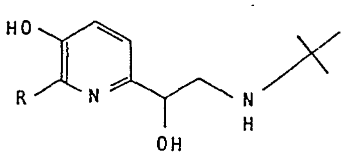

(1)

wherein R is hydrogen, methyl or hydroxymethyl, or a pharmaceutically acceptable acid addition salt thereof, which comprises the hydrogenolysis of an amine of the formula:-

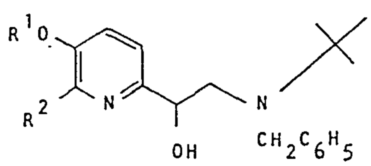

(X)

wherein $R^1$ is benzyl and $R^2$ is hydrogen or methyl, or $R^1$ and $R^2$ are taken together and are 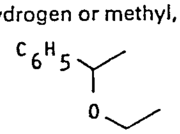 ,

or, alternatively, to prepare compounds of the formula (I) in which R is methyl or hydroxymethyl, the solvolysis followed by the hydrogenolysis of the compound (X) in which $R^1$ and $R^2$ are taken together;

followed by, optionally, converting the product of the formula (I) into a pharmaceutically acceptable acid addition salt by reaction with a suitable acid. Also covered are the novel intermediates of the formula (X). The compound (I) in which R is hydroxymethyl is the well known bronchodilator pirbuterol.

EP 0 058 072 A2

# PROCESS AND INTERMEDIATES FOR PREPARING PIRBUTEROL AND ANALOGS

This invention relates to processes for the preparation of pirbuterol and analogs, i.e. compounds of the formula

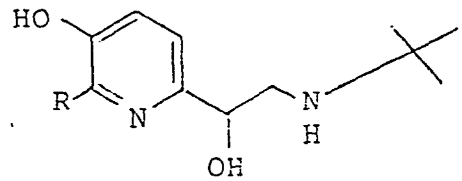

(I)

wherein R is hydrogen, methyl or hydroxymethyl, and certain intermediates therefor. When R is hydroxymethyl, the compound is pirbuterol.

Pirbuterol and its bronchodilator activity were originally described by Barth in a series of U.S. Patents (3,700,681; 3,763,173; 3,772,314; 3,786,160). At that time, pirbuterol was synthesized as follows:

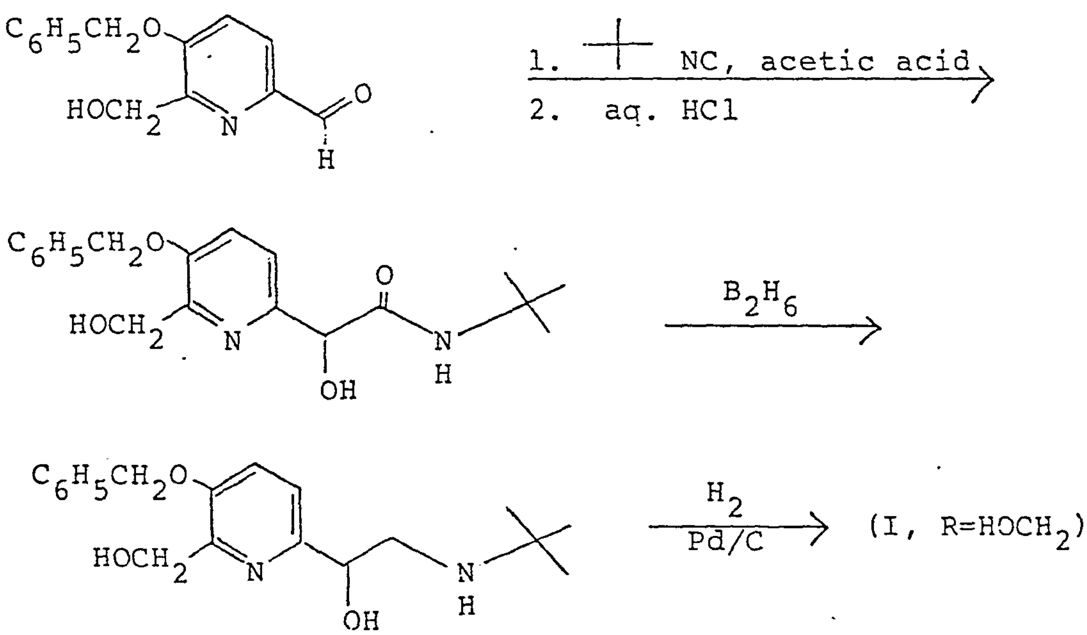

The pirbuterol analogs of the formula I, wherein R is hydrogen, methyl, or methanesulfonylmethyl, and their bronchodilator activity, were disclosed by Jen and Kaiser in U.S. Patent 3,952,101. These compounds were prepared from the corresponding aldehydes by a reaction sequence fully analogous with that of the earlier pirbuterol process.

Subsequently, an alternative, preferred process for the synthesis of pirbuterol was described by Nakanishi (U.S. Patents 3,948,919; 4,031,108), exemplified as follows:

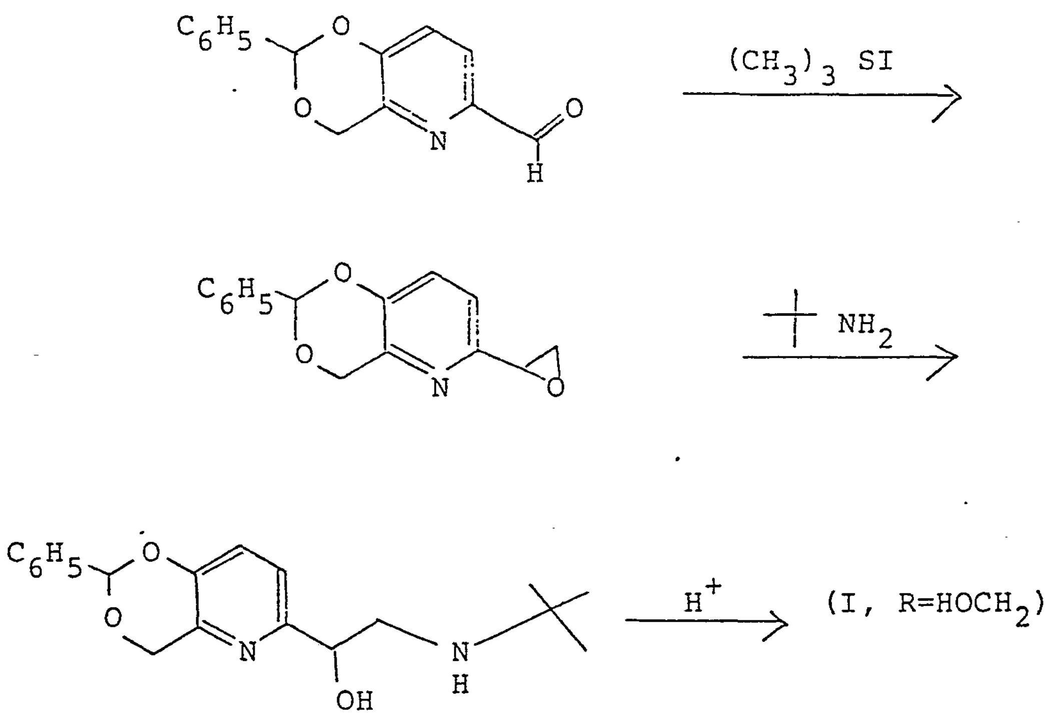


Improvements and alternatives to this process have also been described, viz., isolation of the pirbuterol precursor as a maleate salt (Carroll et al., U.S. Patent 4,011,231) and hydrogenolysis rather than hydrolysis as the final stage (Argentina Patent 214005, Luxembourg Patent 79564).

The latter process still suffers from the disadvantage of generating noxious sulfide by-products in the preparation of epoxide. Even under the best of conditions, traces of sulfur can carry through and increase the catalyst level when protecting groups are removed by hydrogenolysis, the preferred route when the free base form of pirbuterol is desired. Furthermore, the epoxide is relatively unreactive towards the tert-butylamine reagent, even under pressure at elevated temperatures requiring large excess of the reagent and a relatively long time to achieve complete reaction.

A further disadvantage of the improved process is the polar nature of intermediate amino alcohol, making this intermediate difficult to isolate in pure form.

Recently pirbuterol has been discovered to also have utility for the treatment of congestive heart failure (Taylor, U.S. Patent 4,175,128).

In a chemical transformation related to one of the process steps of the present invention, Benderly et al., [Can. J. Chem. 56, pp. 2673-2676 (1978)] have reported the conversion of 2-vinylpyridine to 2-(1,2-epoxyethyl)-pyridine N-oxide by the action of m-chloroperbenzoic acid on 2-vinylpyridine.

The present invention provides a process for the preparation of a compound of the formula:-

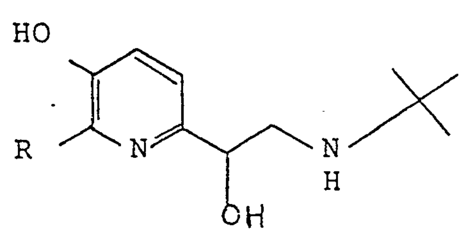

(I)

wherein R is hydrogen, methyl or hydroxymethyl, or a pharmaceutically acceptable acid addition salt thereof, which comprises the hydrogenolysis of an amine of the formula:-

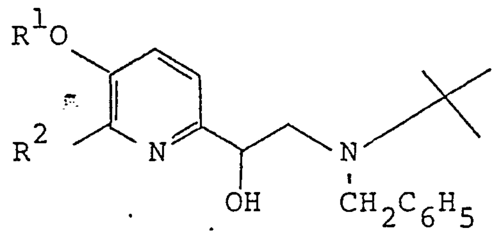

(X)

wherein $R^1$ is benzyl and $R^2$ is hydrogen or methyl, or $R^1$ and $R^2$ are taken together and are 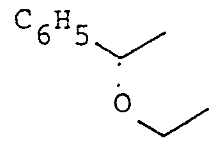 ,

or, alternatively, to prepare compounds of the formula (I) in which R is methyl or hydroxymethyl, the solvolysis followed by the hydrogenolysis of the compound (X) in which $R^1$ and $R^2$ are taken together;

followed by, optionally, converting the product of the formula (I) into a pharmaceutically acceptable acid addition salt by reaction with a suitable acid.

The invention also includes the novel intermediates of the formula (X) above.

The invention also includes the following process:-

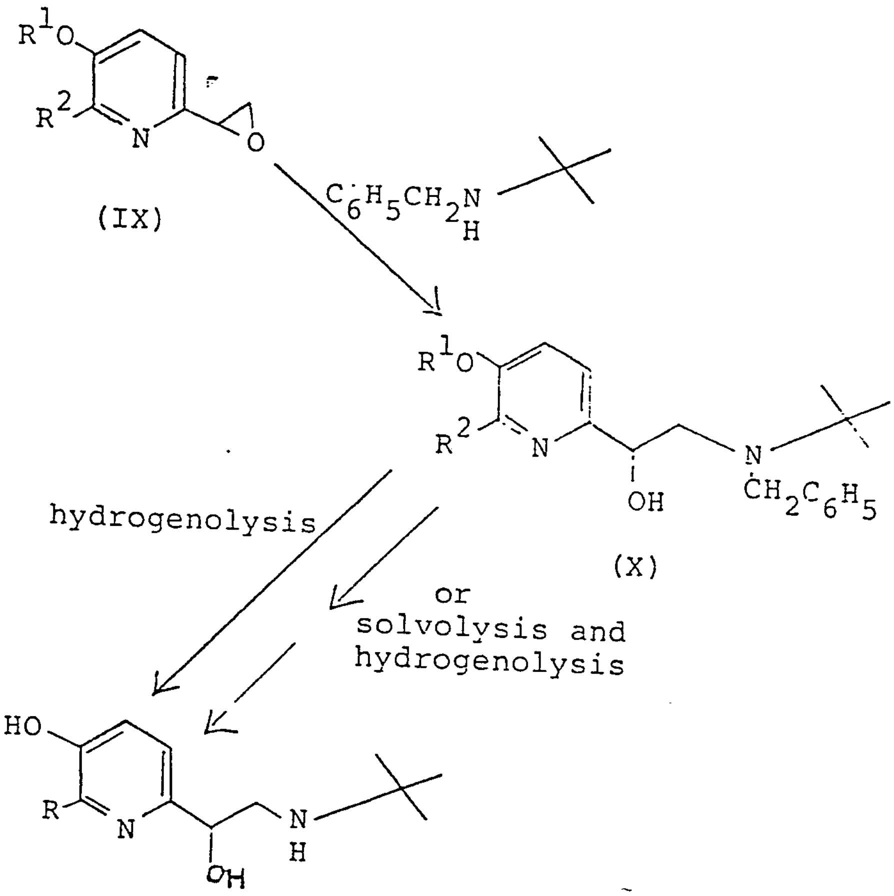


(IX)

(X)

(I)

wherein R is hydrogen, methyl or hydroxymethyl and $R^1$ is benzyl and $R^2$ is hydrogen or methyl; or $R^1$ and $R^2$ are taken together and are 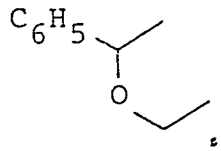.

This process provides a less polar intermediate (X), more readily isolated and purified by standard partition, extraction and crystallization techniques than the corresponding intermediate lacking the benzyl group or the aminoalcohol side chain.

The process of the present invention has been summarized above in flowsheet form. The process is hereinafter described in greater detail, while referring to the flow diagram above.

The conversion of the pyridine-epoxide (IX) to benzylamine (X) is typically accomplished by warming the epoxide with at least one equivalent of N-tert-butylbenzylamine, with or without the presence of a reaction inert diluent. The temperature will generally be elevated (e.g. 50-100°C.) in order to complete the reaction in a reasonable time period. Suitable diluents are ($C_1$-$C_4$) lower alkanols such as methanol, dioxane, 1,2-dimethoxyethane, and the like. Methanol is particularly well suited as diluent, under pressure at a temperature of at or above the atmospheric boiling point of methanol (e.g. 65-80°C.).

In any case, the benzylamine (X) can be hydrogenolyzed under the conditions detailed below to produce the compound I. When $R^1$ and $R^2$ are taken together, the benzylidine group can be first removed by solvolysis, producing

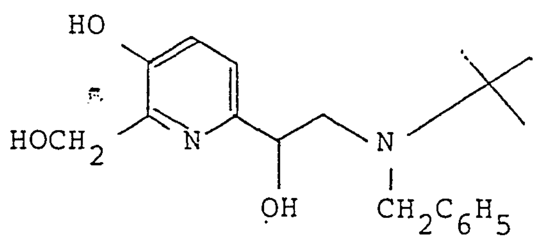

and the benzyl group removed in the final stage of the process by hydrogenolysis.

Hydrogenolyses are accomplished over a noble metal catalyst in a reaction-inert solvent. The temperature of the hydrogenolysis is not critical, e.g. 0-100°C. can be used, but is conveniently carried out at ambient temperature avoiding the costs of cooling or heating. The pressure of the hydrogenation is also not critical (e.g. pressures of subatmospheric to 200 psi or more can be used), but relatively low pressures (e.g. 20-70 psi) are generally sued, since the reaction proceeds at a reasonable rate without requiring the more elaborate equipment characteristic of high pressure hydrogenations. The noble metal catalysts employed in

the present invention include platinum, palladium, ruthenium and rhodium, either of the supported or non-supported type, as well as the known catalytic compounds thereof such as oxides, chlorides, etc. Examples of suitable catalyst supports include carbon and barium sulfate. In the present instance a preferred catalyst is palladium on carbon (1 to 10% of Pd by weight). The hydrogenation solvent is also not critical. It should not react with reactants or product (i.e. it should be reaction inert). Suitable solvents include ($C_1$-$C_4$) alcohols, particularly methanol which can serve as solvent for formation of the hydrochloride salt which is a a common form of pirbuterol. Other solvents such as tetrahydrofuran or dioxane can, of course, be used either alone or in combination with each other and/or the above mentioned alcohols. Higher boiling solvents, (e.g. ethylene glycol, diglyme) can also be used, but are not favored because more energy is required to removed them from the reaction mixture. In order to minimize or avoid hydrogenolysis of the 1-hydroxy-2-tert-butylaminoethyl side chain to a 2-tert-butylethyl group, it is preferred that the hydrogenolysis be carried out in the presence of a small amount of water (e.g. from 1 to 30 molar equivalents). In the particular case of preparing pirbuterol, the reaction is interrupted when the theoretical amount of hydrogen is consumed. If desired hydrogenation can be continued (optimally in the presence of acid, e.g. HCl) so as to carry out the following transformation:

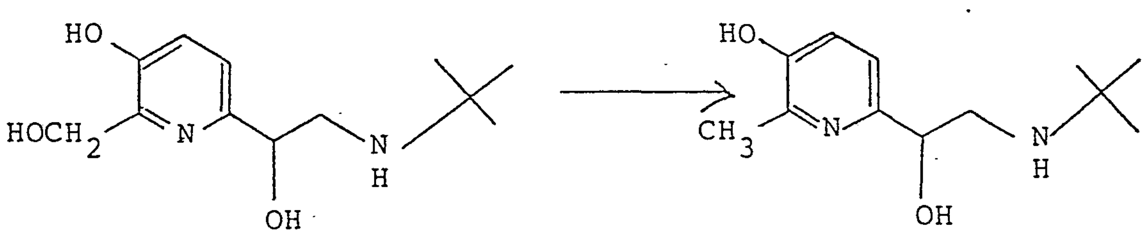

(I, $R=HOCH_2$) (I, $R=CH_3$)

The solvolysis is carried out in aqueous or alcoholic media, with or without cosolvents, usually in the presence of an acid catalyst. Particularly suitable is a combination of methanol and hydrogen chloride from which the intermediate is readily isolated in the form of the hydrochloride salt. Temperature is not critical; e.g. temperature of 0-50°C. or higher can be used, but the reaction is conveniently carried out at ambient temperature. Acid catalyzed solvolysis is preferred over base catalyzed solvolysis, since the products have some tendency to degrade under basic conditions. When solvolysis is used to remove the benzhydryl group, the final stage hydrogenolysis is carried out according to the methods detailed above, preferrably on the free base form compound (XI) in order to avoid, if so desired, over hydrogenation of the 6-hydroxymethyl group to methy.

The present invention is illustrated by the following Example:

EXAMPLE 1

A. 6-[1-Hydroxy-2-(N-*tert*-butylbenzylamino)ethyl]-2-phenyl-4H-1,3-dioxino[5,4-b]pyridine

A solution of 33.0 g. (0.13 mole) 6-(1,2-epoxyethyl)-2-phenyl-4H-1,3-dioxino[5,4-b]pyridine and 22.0 g. (0.135 mole) of N-*tert*-butylbenzylamine in 200 ml. of methanol was heated to the reflux temperature overnight. Upon cooling there deposited 6-[1-hydroxy-2-(N-*tert*-butylbenzylamino)ethyl]pyridine which was recrystallized from isopropanol to give 29.4 g. (54%) of the amino alcohol m.p. 126-130°C.

Anal. Calcd. for $C_{26}H_{30}N_2O_3$: C, 74.61; H, 7.23; N, 6.69

Found : C, 75.04; H, 7.21; N, 7.15

B. 2-Hydroxymethyl-3-hydroxy-6-[1-hydroxy-2-(N-*tert*-butylbenzylamino)ethyl]pyridine Dihydrochloride

A solution of 8.40 g. (0.02 mole) 6-[1-hydroxy-2-(N-*tert*-butylbenzylamino)ethyl]-2-phenyl-4H-1,3-dioxino[5,4-b]pyridine in 75 ml. of methanol containing 10 ml. of concentrated hydrochloric acid was stirred at room temperature for 4 hours, then the solvents were evaporated *in vacuo* to give a solid. Recrystallization from isopropanol/methanol gave 6.30 g. (74%) of 2-hydroxyethyl-3-hydroxy-6-[1-hydroxy-2-(N-*tert*-butylbenzylamino)ethyl]pyridine dihydrochloride; m.p. 173-175°C. (dec.)

Anal. Calcd. for $C_{19}H_{26}N_2O_3.2HCl.1/2H_2O$:

C, 55.34; H, 7.09; N, 6.80

Found: C, 55.73; H, 7.28; N, 6.90

C. Pirbuterol Hydrochloride

In a Parr bottle (250 ml.) there were placed 0.50 g. (0.0012 mole) of 6-[1-hydroxy-2-(N-*tert*-butylbenzylamino)ethyl]-2-phenyl-4H-1,3-dioxino[5,4-b]-pyridine and 500 mg. of 5% palladium on carbon in 20 ml. of methanol. Under a hydrogen pressure of 50 psi the mixture was reacted at room temperature for 2 hours. The catalyst was removed by filtration under a nitrogen atmosphere and the solvent was saturated with dry hydrogen chloride to give pirbuterol hydrochloride 250 mg. (67%); m.p. 174-175° dec.

CLAIMS

1. A process for the preparation of a compound of the formula:-

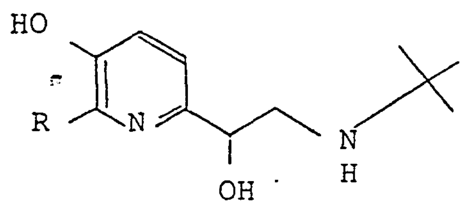

(I)

wherein R is hydrogen, methyl or hydroxymethyl, or a pharmaceutically acceptable acid addition salt thereof, which comprises the hydrogenolysis of an amine of the formula:-

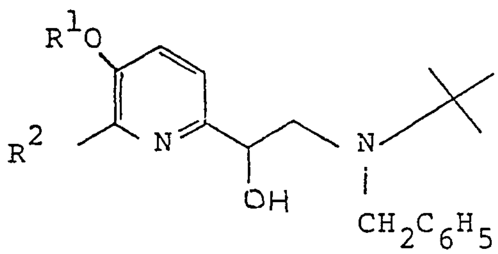

(X)

wherein $R^1$ is benzyl and $R^2$ is hydrogen or methyl, or $R^1$ and $R^2$ are taken together and are $C_6H_5$ 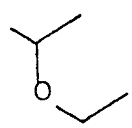 ;

or, alternatively, to prepare compounds of the formula (I) in which R is methyl or hydroxymethyl, the solvolysis followed by the hydrogenolysis of the compound (X) in which $R^1$ and $R^2$ are taken together;
followed by, optionally, converting the product of the formula (I) into a pharmaceutically acceptable acid addition salt by reaction with a suitable acid.

2. A process according to claim 1, characterised in that the compound (VIII) is prepared by reacting N-tert-butylbenzylamine with an epoxide of the formula

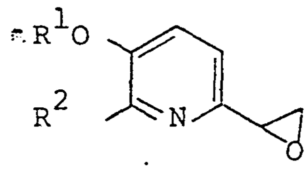

(IX)

wherein $R^1$ and $R^2$ are as defined in claim 1.

3. A process according to claim 1 or 2, wherein the solvolysis is carried out with a combination of methanol and hydrochloric acid and the hydrogenolysis is carried out using hydrogen over a noble metal catalyst.

4. A process according to any one of the preceding claims wherein $R^1$ and $R^2$ are taken together and are

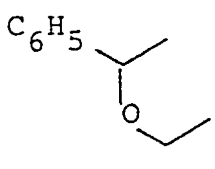 ,

and wherein the compound (I) in which R is hydroxymethyl is produced.

5. A compound of the formula

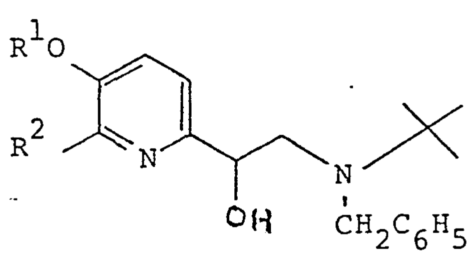

(X)

wherein $R^1$ is benzyl and $R^2$ is hydrogen or methyl; or $R^1$ and $R^2$ are taken together and are 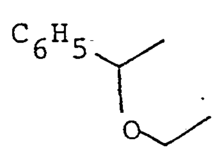 .

6. The compound of claim 5 wherein $R^1$ and $R^2$ are taken together and are $C_6H_5$ 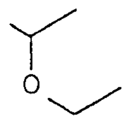 .

CLAIMS FOR AUSTRIA

1. A process for the preparation of a compound of the formula:-

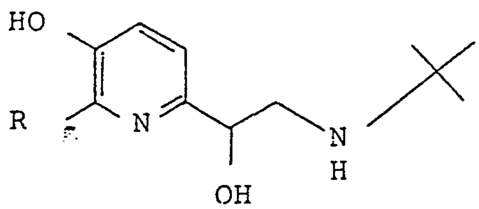

(I)

wherein R is hydrogen, methyl or hydroxymethyl, or a pharmaceutically acceptable acid addition salt thereof, which comprises the hydrogenolysis of an amine of the formula:-

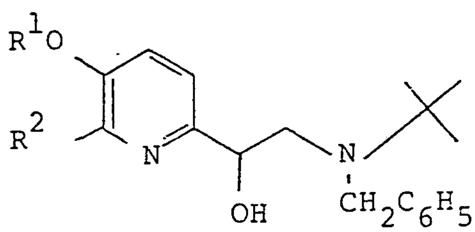

(X)

wherein $R^1$ is benzyl and $R^2$ is hydrogen or methyl, or $R^1$ and $R^2$ are taken together and are 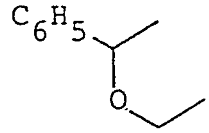 ,

or, alternatively, to prepare compounds of the formula (I) in which R is methyl or hydroxymethyl, the solvolysis followed by the hydrogenolysis of the compound (X) in which $R^1$ and $R^2$ are taken together;

followed by, optionally, converting the product of the formula (I) into a pharmaceutically acceptable acid addition salt by reaction with a suitable acid.

2. A process according to claim 1, characterised in that the compound (VIII) is prepared by reacting N-tert-butylbenzylamine with an epoxide of the formula

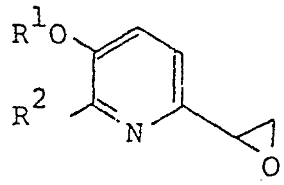

(IX)

wherein $R^1$ and $R^2$ are as defined in claim 1.

3. A process according to claim 1 or 2, wherein the solvolysis is carried out with a combination of methanol and hydrochloric acid and the hydrogenolysis is carried out using hydrogen over a noble metal catalyst.

4. A process according to any one of the preceding claims wherein $R^1$ and $R^2$ are taken together and are 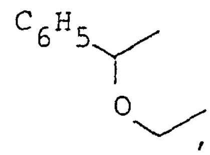,

and wherein the compound (I) in which R is hydroxymethyl is produced.